# EUROPEAN PATENT SPECIFICATION

(11) **EP 4 628 740 B1**
(45) Date of publication and mention of the grant of the patent: **07.10.2026**
(21) Application number: 23896615.4
(22) Date of filing: 22.11.2023
(51) Int. Cl.: F16C 11/10, B67D 3/00, A61M 5/14, B67D 7/06, B67D 7/08, B67D 7/32, B67D 7/58, G01D 21/02, F04B 43/12, F04B 53/16

(54) **LIQUID TRANSFERRING DEVICE**
FLÜSSIGKEITSÜBERTRAGUNGSVORRICHTUNG
DISPOSITIF DE TRANSFERT DE LIQUIDE

(30) Priority: 30.11.2022 CN 202211519527
(43) Date of publication of application: 08.10.2025
(73) Proprietor: Shenzhen Cellbri Bio-Innovation Technology Co., Ltd., Shenzhen, Guangdong 518107 (CN)
(72) Inventor: HUANG, Minzhong, Shenzhen, Guangdong 518107 (CN); YE, Peichen, Shenzhen, Guangdong 518107 (CN); FANG, Rulin, Shenzhen, Guangdong 518107 (CN); LIU, Fujing, Shenzhen, Guangdong 518107 (CN); SHANG, Yuanfang, Shenzhen, Guangdong 518107 (CN); GUO, Xiaoliang, Shenzhen, Guangdong 518107 (CN)
(74) Representative: Herrero & Asociados, S.L.
(86) International application number: PCT/CN2023/133112
(87) International publication number: WO 2024/114464

(56) References cited:
- WO-A1-2022/081519
- WO-A1-2022/081519
- CN-A- 111 939 379
- CN-A- 111 939 379
- CN-U- 207 666 932
- CN-U- 207 666 932
- CN-U- 208 852 131
- CN-U- 208 852 131
- CN-Y- 2 649 097
- US-A1- 2020 023 158

## Description

### Technical Field

The application belongs to the technical field of liquid transfer, in particular to a liquid transfer device.

### Background

Recently, the inventor(s) discovered that liquid transfer devices (such as fully enclosed liquid transfer instruments) are generally bulky and difficult to move, and must be positioned in a permanent position with large space for liquid transfer operation, which is inconvenient to use. Moreover, the existing liquid transfer device often lacks a built-in battery and requires an external power supply to function. The power cord limits its mobility and functionality, reducing the user experience.

In the prior art, WO 2022/081519 A1 discloses a housing-based liquid processing device including pumps, valves, and a pivotable platform driven by a rocker mechanism, which is mainly intended for stationary laboratory use and does not address compact storage or improved portability. CN 207 666 932 U discloses a liquid fusion device with a foldable and stretchable support structure for hanging liquid bags, focusing on space reduction during storage, but it does not disclose a fully enclosed liquid transfer device integrating a compact housing and a rotating self-locking support structure. CN 111 939 379 A discloses a high-pressure injection auxiliary device with liquid level monitoring and pipeline control, but it is primarily directed to injection assistance and does not address portability or flexible positioning of a liquid transfer device.

### Summary of the Invention

The present application provides a retractable and foldable liquid transfer device that is small in size. And the portability of the liquid transfer device is improved.

In view of the above technical problems, an embodiment of the present application provides a liquid transfer device as defined by the subject-matter of claim 1.

The liquid transfer device of the application can realize liquid flow to transfer liquid through the liquid path mechanism. Meanwhile, the telescopic structure of the application can be extended or shortened. The rotating self-locking structure can rotate and fold the telescopic structure, and lock it in place at a preset position (ensuring the stability of the liquid transfer operation of the device). Therefore, with the above-mentioned telescopic structure and rotating self-locking structure, the liquid transfer operation of the liquid transfer device can be ensured, while the volume of the liquid transfer device is also reduced. The liquid transfer device in the application is small in size and light in weight. When the telescopic structure is completely retracted and driven to be folded by the rotating self-locking structure, the length, width and height of the liquid transfer device are 300 mm*220 mm*150 mm. It can be easily moved or placed into a biosafety cabinet or a clean workbench for use by just one person. Therefore, the liquid transfer device offered by the current application enhances its portability, guarantees regular liquid transfer operation, and also improves its structural stability and ease of mobility, making it easy for users to use the device in a small space.

The following figures and descriptions provide details for one or more embodiments of the application, and other features and advantages of the application will be apparent from the description, drawings and claims.

### Brief description of the drawings

The application will be further illustrated with the drawings and embodiments.
Fig. 1 is a structural schematic diagram of the liquid transfer device according to an embodiment of the present application.
Fig. 2 is a structural schematic diagram of the electrical assembly of the liquid transfer device according to an embodiment of the present application.
Fig. 3 is a structural schematic diagram of the housing assembly of the liquid transfer device according to an embodiment of the present application.
Fig. 4 is a schematic diagram of the installation structure of the bubble sensor and driving member of the liquid transfer device according to an embodiment of the present application.
Fig. 5 is a structural schematic diagram of the liquid transfer device according to an embodiment of the present application when the telescopic structure is in an unfolded state. In this state, the rotating self-locking structure rotates to 90°and locks the telescopic structure.
Fig. 6 is a structural schematic diagram of the liquid transfer device according to an embodiment of the present application when the telescopic structure is in a retracted state. In this state, the rotating self-locking structure rotates to 0°and locks the telescopic structure.
Fig. 7 is a structural schematic diagram of the telescopic structure of the liquid transfer device according to an embodiment of the present application.
Fig. 8 is an explosive view of the telescopic structure of the liquid transfer device according to an embodiment of the present application.
Fig. 9 is a structural schematic diagram of the sliding block of the telescopic structure according to an embodiment of the present application.
Fig. 10 is a structural schematic diagram when the sliding plate of the telescopic structure is retracted in the sliding space and the clamping groove is clamped with the resisting buckle according to an embodiment of the application.
Fig. 11 is a structural schematic diagram when the sliding block of the telescopic structure slides upward with the sliding plate until the clamping groove is no longer clamped with the resisting buckle, according to an embodiment of the present application
Fig. 12 is a structural schematic diagram when the sliding block of the telescopic structure is no longer clamped with the resisting buckle, and the sliding plate slides along the slide rail until the positioning assembly and the positioning hole are locked, according to an embodiment of the present application
Fig. 13 is a structural schematic diagram when the sliding block of the telescopic structure slides upward with the sliding plate until it is out of the positioning hole, and the clamping groove has not been clamped with the resisting buckle, according to an embodiment of the present application
Fig. 14 is a structural schematic diagram when the sliding block of the telescopic structure slides down with the sliding plate until the clamping groove is clamped with the resisting buckle, according to an embodiment of the present application.
Fig. 15 is an explosive view of the rotating self-locking structure of the liquid transfer device according to an embodiment of the present application.
Fig. 16 is a structural schematic diagram in which the locking column of the rotating self-locking structure slides into the first positioning groove according to an embodiment of the present application.
Fig. 17 is a structural schematic diagram in which the locking column of the rotating self-locking structure slides out of the first positioning groove according to an embodiment of the present application.
Fig. 18 is a structural schematic diagram in which the locking column of the rotating self-locking structure slides into the first positioning groove and the arc buffer abuts against the stop block, according to an embodiment of the present application.
Fig. 19 is a structural schematic diagram in which the locking column of the rotating self-locking structure slides into the second positioning groove according to an embodiment of the present application.
Fig. 20 is a structural schematic diagram in which the locking column of the rotating self-locking structure slides into the second positioning groove and the arc buffer abuts against the stop block, according to an embodiment of the present application.

Reference signs in the drawings are as follows.

100. Liquid path mechanism; 110. Conduit assembly; 120. Driving member; 121. Pump head. 122. First gasket; 123. Pump shaft; 124. Pump support; 125. Motor; 130. Bubble sensor; 131. Second gasket; 140. Liquid path mounting plate; 150. Liquid path support rod; 200. Housing assembly; 210. Bottom plate; 220. Housing; 221. Accommodating space; 222. Main device housing; 223. Back plate; 224. Bottom pad; 300. Support frame; 310. Telescopic structure; 10. Support assembly; 101. Support plate; 1011. Sliding space; 1012. Upper plate; 1013. Lower plate; 102. Slide rail; 103. Positioning hole; 20. Sliding assembly; 201. Sliding plate; 202. Positioning assembly; 2021. Mounting hole; 2022. First spring; 2023. Sliding block; 20231. Resisting surface; 20232. Guide surface; 20233. Clamping groove; 20234. Guide groove; 2024. Positioning pin; 2025. Resisting buckle; 20251. Through hole; 2026. Guide rail; 203. Sliding wheel; 204. Front cover; 205. Back cover; 30. Top support block; 301. Hook; 302. Placement hole; 303. Telescopic opening; 320. Rotating self-locking structure; 1. Rotating assembly; 11. First rotating shaft; 111. Positioning groove; 112. Connecting segment; 113. Cooperating segment; 114. Accommodating groove; 115. Arc baffle; 12. Second rotating shaft; 2. Bracket; 21. First sliding hole; 22. Second sliding hole; 23. Sliding groove; 24. Accommodating hole; 25. Stop block; 26. Bearing hole; 28. First support plate; 29. Second support plate; 3. Lock assembly; 31. Elastic self-locking member; 311. Locking column; 312. Lock buckle; 3121. Sliding column; 313. Second spring; 314. Unlocking pin; 32. Torque hinge; 4. Bearing; 400. Electrical assembly; 410. Control assembly; 420. Switch button; 430. Battery; 440. Power supply interface; 450. Touch screen; 500. Liquid bag.

### Detailed description of disclosed embodiments

In order to make the technical problems, technical solutions and beneficial effects of the present application more clear, the application will be further explained in detail below with reference to the drawings and embodiments. It should be understood that the specific embodiments described here are only used to illustrate the application, rather than to limit the application.

It should be understood that the orientations or positional relationships indicated by the terms "upper", "lower", "left", "right", "front", "rear" and "middle" and the like are based on the orientations or positional relationships shown in the attached drawings, only for convenience of describing the present invention and simplifying the description, and do not indicate or imply that the said device or element must have a specific orientation, be constructed or operated in a particular orientation, and therefore cannot be understood as a limitation of the present application.

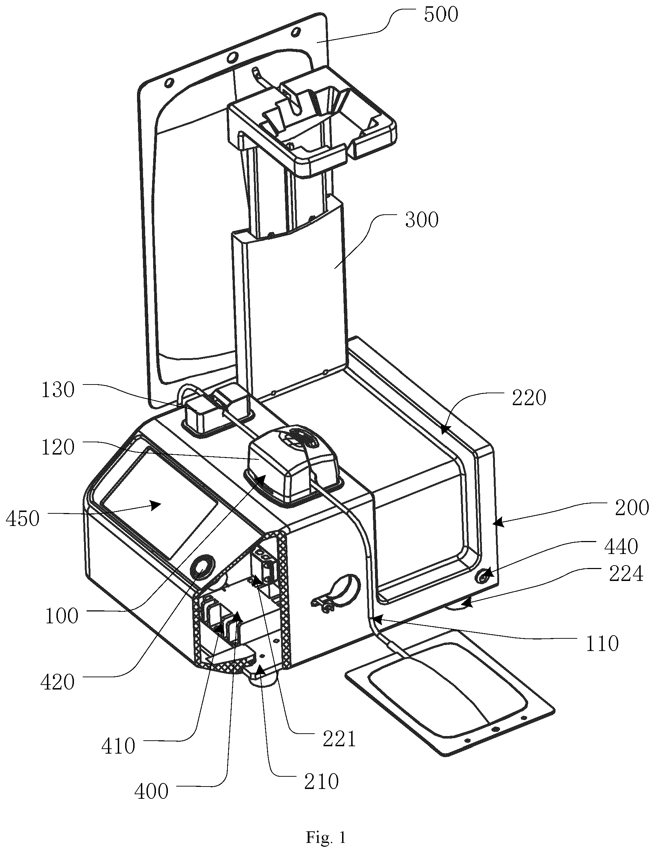

As shown in Figs. 1 to 6, an embodiment of the present application provides a liquid transfer device, including the following components.

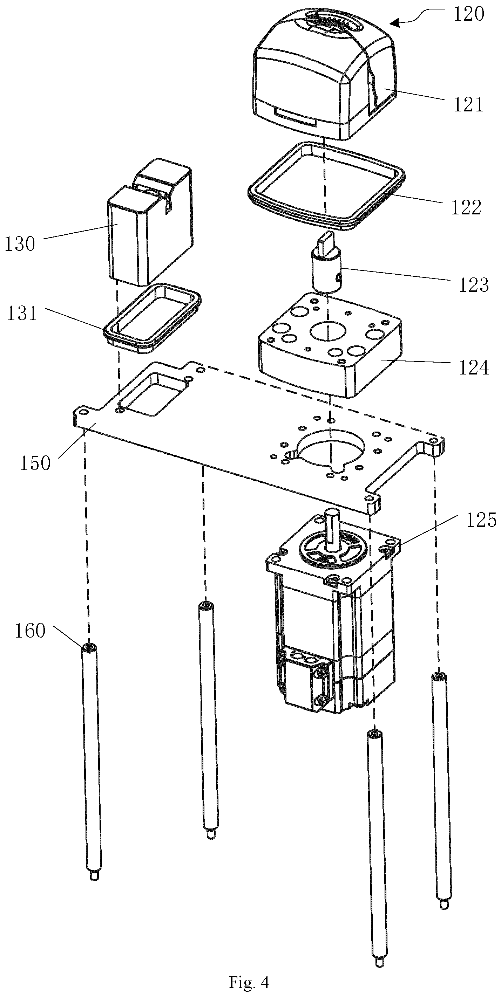

A liquid path mechanism 100, which includes a conduit assembly 110 and a driving member 120 for driving liquid to flow in the conduit assembly 110. Optionally, as shown in Fig. 1, the liquid path mechanism 100 is also installed on the bubble sensor 130 on the housing 220, and the bubble sensor 130 is used to detect the liquid flow state in the conduit assembly 110. Specifically, as shown in Fig. 4, the driving member 120 includes, but is not limited to, a peristaltic pump. The pump support 2124 is fixed on the liquid path mounting plate 140 by screw connection, and the pump head 121 of the peristaltic pump is fixed on the pump support 2124 by screw connection. One end of the pump shaft 123 of the peristaltic pump is connected with the output shaft sleeve of the motor 125 and fixed on the output shaft of the motor 125 via a jackscrew. In this state, the pump shaft 123 can rotate together with the output shaft of the motor 125, and the motor 125 is fixed on the liquid path mounting plate 140 via threaded connection. Moreover, the end of the pump shaft 123 away from the motor 125 should be clamped into the groove at the bottom of the pump head 121 of the peristaltic pump, that is, the pump shaft 123 can rotate together with the output shaft of the motor 125, and then drive the peristaltic pump to rotate to squeeze the liquid flow in the conduit assembly 110, so as to realize the liquid transfer. While the bubble sensor 130 is fixed on the liquid path mounting plate 140 via threaded connection, understandably, the bubble sensor 130 is used to monitor whether bubbles are mixed in the liquid in the conduit assembly 110 in real time, and then determine the liquid flow state. For example, the bubble sensor 130 detects that there are multiple bubbles in the liquid flowing in the conduit assembly 110 for one second, which indicates that no liquid has flowed in the conduit assembly 110. This suggests that the liquid to be transferred has been exhausted (or will soon be exhausted). Furthermore, the liquid path mechanism 100 also includes a pressure sensor, which can detect the pressure of the liquid in the conduit assembly 110 to obtain a pressure detection result. In this application, the liquid transfer state can be determined according to the liquid flow state and pressure detection results, and then instructions such as stopping liquid transfer command and alarm can be issued according to the liquid transfer state.

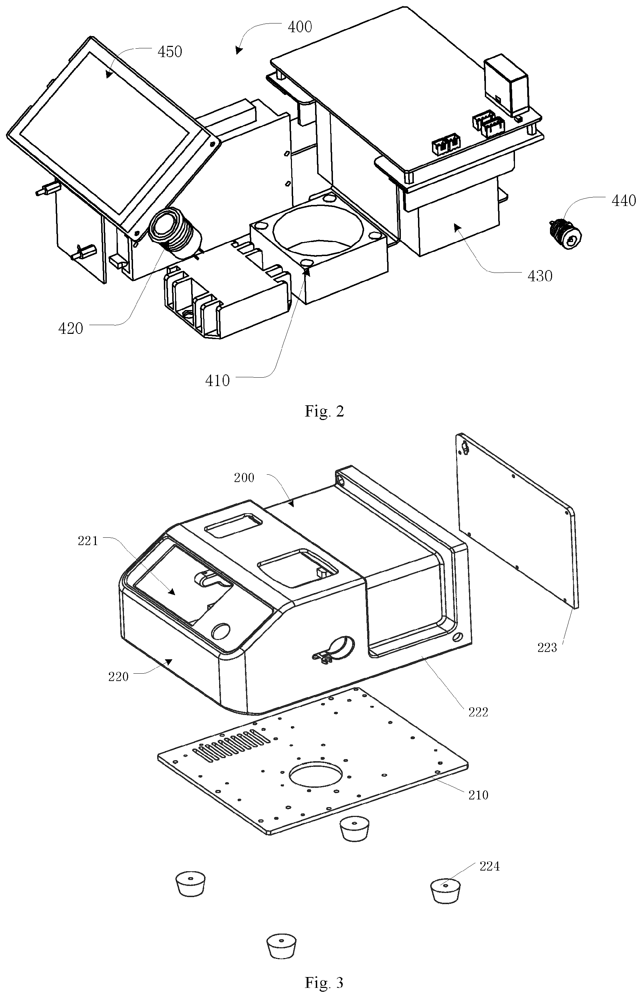

A housing assembly 200, which includes a bottom plate 210 and a housing 220 with an accommodating space 221, and both the conduit assembly 110 and the driving member 120 are installed on the housing 220. Understandably, as shown in Fig. 4, the liquid path mounting plate 140 is fixed on the bottom plate 210 via the liquid path support rod 150, allowing the installation structure of the liquid path mechanism 100 to be more stable. The housing 220 is provided with a first through hole for allowing the pump head 121 of the peristaltic pump to extend from the accommodating space 221 and clamp the conduit assembly 110. The pump head 121 and the housing 220 are connected sealingly via the first gasket 122 to prevent water, oil or other impurities from entering the accommodating space 221 from the installation position between them. Furthermore, the housing 220 is provided with a second through hole for enabling the bubble sensor 130 to extend out of the accommodating space 221 and clamp the conduit assembly 110 for alignment, so as to measure the liquid flow state. And the bubble sensor 130 and the housing 220 are connected sealingly via the second gasket 131 to prevent water, oil and other impurities from entering the accommodating space 221 from the installation position between them. Optionally, as shown in Fig. 3, the housing 220 includes a main device housing 222 installed on the bottom plate 210 and having an opening, and a back plate 223 installed at the opening position of the main device housing 222 and connected to the back plate 223 of the bottom plate 210. The main device housing 222, back plate 223 and bottom plate 210 enclose to form the accommodating space 221, and the control assembly 410 is installed on the bottom plate 210 and located in the accommodating space 221. In the embodiment shown in Fig. 3, the housing 220 further includes a bottom pad 224, and the bottom pad 224 is fixed on the bottom of the bottom plate 210 via threaded connection, and the main device housing 222 is fixed on the bottom plate 210 via threaded connection. The back plate 223 is fixed on main device housing 222 and bottom plate 210 via threaded connection, and the housing assembly 200 is used to support and protect the whole liquid transfer device.

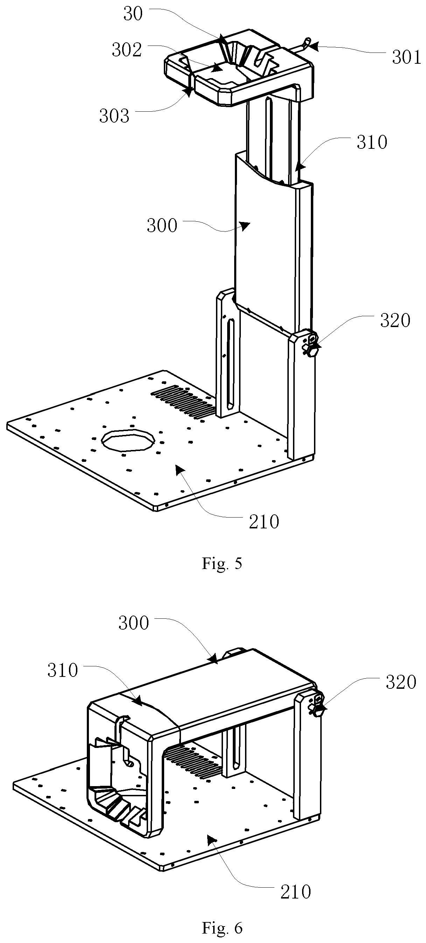

A support frame 300, which includes a rotating self-locking structure 320 and a telescopic structure 310. As shown in Figs. 5 and 6, the telescopic structure 310 is installed on the bottom plate 210 through the rotating self-locking structure 320. The rotating self-locking structure 320 is used to drive the telescopic structure 310 to rotate within a preset angle range relative to the bottom plate 210, and lock the telescopic structure 310 to stop rotating when it rotates to a preset position. Specifically, the support frame 300 is mainly used for placing a liquid bag 500 or a bottle (such as a bottle made of plastic, glass, etc.) that needs liquid transfer, and the shapes of the liquid bag 500 and the bottle are not restricted. The telescopic and foldable rotating self-locking structure 320, which is composed of the telescopic structure 310 and the rotating self-locking structure 320, is designed to reduce the device's volume while also enabling the liquid transfer device to operate conveniently.

An electrical assembly 400, which includes a control assembly 410 installed in the accommodating space 221, and the control assembly 410 is connected with the driving member 120. As shown in Figs. 1 and 2, the electrical assembly 400 is fixedly installed on the housing assembly 200, and the control assembly 410 in the electrical assembly 400 is used to control the operation of device. For example, the control assembly 410 is connected with the driving member 120, the bubble sensor 130, the pressure sensor, etc., and is used to control the operation of various components. Optionally, the electrical assembly 400 further includes a touch screen 450 installed on the housing 220 and connected to the control assembly 410. The touch screen 450 is a man-machine interactive interface, which can be used for parameter adjustment and instruction operation, etc. The touch screen 450 is connected with the control assembly 410, so that the liquid transfer parameters and liquid transfer modes can be set via the touch screen 450, and then different liquid transfer operations can be performed through the controller according to the above settings.

Furthermore, as shown in Figs. 1 and 2, the electrical assembly 400 further includes a switch button 420 installed on the housing 220 and connected to the control assembly 410. In this way, with the trigger of the switch button 420, the controller can start or turn off the liquid transfer device. Optionally, the electrical assembly 400 further includes a battery 430 connected to the control assembly 410 and a power supply interface 440 connected to the battery 430 and the control assembly 410. Specifically, the battery 430 can supply power to the liquid transfer device when the power supply is not turned on. The liquid transfer device of this application has a built-in battery 430, which can enable the liquid transfer device to work for 48 hours in the standby state and continuously transfer liquid for 4 hours in the liquid transfer state. In this application, the power supply interface 440 can be connected with an external power supply to supply power to the liquid transfer device, and can also charge the battery 430. Understandably, the electrical assembly 400 of the present application further includes a prompting device connected to the control assembly 410, which may be a buzzer that emits a prompting sound, prompts when the liquid transfer is complete, or is used when an abnormal alarm is triggered. Understandably, the prompting device may also be a light prompting device capable of emitting lights with different colors and flashing frequencies, as well as performing various sorts of light prompting under varying prompting conditions.

The liquid transfer device of the present application can realize liquid flow to transfer liquid through the liquid path mechanism 100. Meanwhile, the telescopic structure 310 of the present application can be lengthened or shortened, and the rotating self-locking structure 320 can rotate and fold the telescopic structure 310, and lock it at the preset position (ensuring the stability of the device for liquid transfer operation). Furthermore, with the above-mentioned telescopic structure 310 and rotating self-locking structure 320, the volume of the liquid transfer device can be reduced while assuring that it can conduct liquid transfer operations. The liquid transfer device in this application is small in size and light in weight. When the telescopic structure 310 is completely retracted and is folded by the rotating self-locking structure 320, the size, length, width and height of the liquid transfer device are 300 mm*220 mm*150 mm, which can be easily moved by a single person or put into a biosafety cabinet or a clean workbench for operation. Therefore, the liquid transfer device provided by the present application improves the portability of the liquid transfer device, ensures the normal liquid transfer operation, and also improves the moving convenience and structural stability, which is convenient for users to use the liquid transfer device in a small space. The liquid transfer device in this application can automatically transfer the liquid in the bottle or the liquid bag 500 to the targeted container through the liquid path mechanism 100. The flow speed range of the liquid transfer device in this application is 0-200 ml/min, and the volume of the transferred liquid can be set as required.

The working principle of the liquid transfer device in an embodiment of the application is as follows. When the liquid transfer device is powered on or the battery 430 has enough power, press the switch button 420, wait for a few seconds, and the liquid transfer device will turn on and finish the self-test, allowing regular functioning to resume. In this state, as shown in Fig. 1, a large liquid bag 500 with liquid to be transferred is hung on the support frame 300 (or a bottle with liquid to be transferred is placed in the placement hole 302 of the top support block 30 at the top of the support frame 300. Understandably, as shown in Fig. 5, the top of the placement hole 302 is provided with an inclined plane, so that the opening of the inverted bottle can fit and fit with the transition inclined plane of the bottle body, and the installation is stable. And the placement hole 302 has a telescopic opening 303, and the aperture size of the placement hole 302 can be adjusted within a certain range through the setting of the telescopic opening, so as to be suitable for bottles of different sizes). Then the conduit assembly 110 is clamped into the bubble sensor 130 and the pump head 121 of the peristaltic pump, and the target container (such as a small liquid bag) connected to the conduit assembly 110 is placed in a preset position next to the liquid transfer device. Then, select the volume and speed of liquid transfer on the operation interface of the touch screen 450. And then click "Start" on the touch screen 450, the liquid transfer device will start to transfer the liquid in the large liquid bag 500 into the small liquid bag. When the buzzer on the liquid transfer device makes the "didi" sound, it indicates that the liquid transfer is complete.

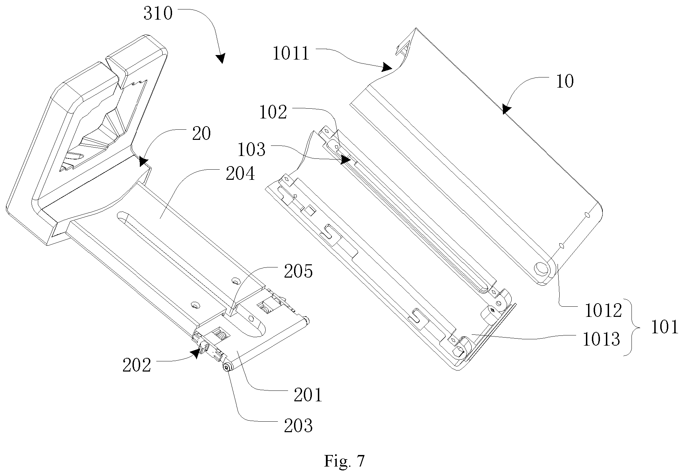

In an embodiment, as shown in Figs. 7 to 9, the telescopic structure 310 includes the following components.

A support assembly 10, which includes a support plate 101 with a sliding space 1011, a slide rail 102 arranged on the inner side wall of the sliding space 1011, and a positioning hole 103 arranged on the slide rail 102 and communicating with the sliding space 1011. Optionally, the support plate 101 includes an upper plate 1012 and a lower plate 1013 connected to the upper plate 1012, and the sliding space 1011 is formed between the upper plate 1012 and the lower plate 1013. The upper plate 1012 and the lower plate 1013 can be fixedly connected by snap connection, screw connection, welding and the like, or the upper plate 1012 and the lower plate 1013 can be integrally formed.

A sliding assembly 20, which includes a sliding plate 201 arranged in the sliding space 1011 and sliding telescopically along the slide rail 102, and a positioning assembly 202 arranged on the sliding plate 201. The positioning assembly 202 is used for locking with the positioning hole 103 when the sliding plate 201 slides towards the first end of the slide rail 102 to stop the sliding of the sliding plate 201. The positioning assembly 202 is also used to unlock the positioning hole 103 when the sliding plate 201 slides towards the second end of the slide rail 102.

As shown in Fig. 7, the support assembly 10 includes two slide rails 102 arranged on opposite sides of the sliding plate 201. The sliding plate 201 slides along the slide rail 102 arranged on both sides of the sliding plate 201 to realize the telescopic movement between the sliding plate 201 and the support plate 101. The sliding assembly 20 includes two positioning assemblies 202 arranged on opposite sides of the sliding plate 201. The positioning holes 103 are symmetrically arranged on two slide rails 102. The positioning assemblies 202 are symmetrically arranged on the opposite sides of the sliding plate 201, so as to cooperate with the positioning hole 103 to lock or unlock the sliding plate 201. Furthermore, the sliding assembly 20 further includes a front cover 204 and a back cover 205 respectively installed on opposite sides of the sliding plate 201. The front cover 204, the sliding plate 201 and the back cover 205 are fixedly connected. Specifically, the front cover 204 and back cover 205 can be fixedly connected by means of snap connection, screw connection, welding and the like. Or the front cover 204 and the back cover 205 can be integrally formed. However, the sliding plate 201 needs to be fixedly installed between the front cover 204 and the back cover 205 (for example, the sliding plate 201 can be fixed on the back cover 205 by screws). The sliding plate 201, front cover 204 or back cover 205 can be arranged with a top support block 30. Components required for operations such as liquid transfer can be installed on the top support block 30 (such as the hook 301 required when the liquid bag 500 for liquid transfer is hung, such as the hook 301 of the retractable and foldable support frame 300 shown in Fig. 7, with a limit load of 5 kg). And as shown in Fig. 5, the top support block 30 is provided with a placement hole 302 with a telescopic opening 303, and the top support block 30 can be designed with the same height as the first support plate 28 and the second support plate 29, so as to enable the top support block 30, first support plate 28 and second support plate 29 to jointly support the locked telescopic structure 310 when the telescopic structure 310 rotates to 0 degrees as shown in Fig. 6. Furthermore, after the support plate 101 is installed on the bottom plate 210, the components installed on the top support block 30 can be driven to move synchronously by the movement of the sliding plate 201 relative to the support plate 101, and then the distance between the bottom plate 210 and the components can be adjusted.

The telescopic structure 310 of this embodiment allows for a length expansion adjustment between the sliding plate 201 and support plate 101. Specifically, the sliding plate 201 of the telescopic structure 310 can be shrunk in the sliding space 1011 of the support plate 101, thereby reducing the size of the whole liquid transfer device. However, when the length between the movable plate and the support plate 101 needs to be adjusted according to the needs of the operation related to liquid transfer, the sliding plate 201 retracted in the sliding space 1011 slides along the slide rail 102. In this state, the length between the sliding plate 201 and the support plate 101 is extended. And when the sliding plate 201 slides to the positioning assembly 202 and locks with the positioning hole 103, the sliding plate 201 can be fixed at this position, thus ensuring the structural stability of the liquid transfer device when performing operations such as liquid transfer. In this application, the telescopic structure 310 enables the combined length of the sliding plate 201 and supporting plate 101 to be extended to a preset length (after retracted, the length of the sliding plate 201 and supporting plate 101 can be reduced by about half, and the preset length may be 100 mm), and the locking can be realized by the positioning hole 103 and the positioning assembly 202 at the preset length. The telescopic structure 310 of the present application improves the portability and structural stability of the liquid transfer device, and improves its moving convenience while ensuring regular liquid transfer operations, which is also convenient for users to use it for liquid transfer operation in a small space.

In an embodiment, as shown in Figs. 7 to 8, the sliding assembly 20 further includes a sliding wheel 203 arranged on the sliding plate 201, and the sliding plate 201 slides along the slide rail 102 in a telescopic manner through the sliding wheel 203. That is, the two sliding wheels 203 are respectively fixed on the opposite sides of the sliding plate 201 by screws, and the two sliding wheels 203 can slide along the two slide rail 102 at the same time, thereby driving the sliding plate 201 to move smoothly in the sliding space 1011.

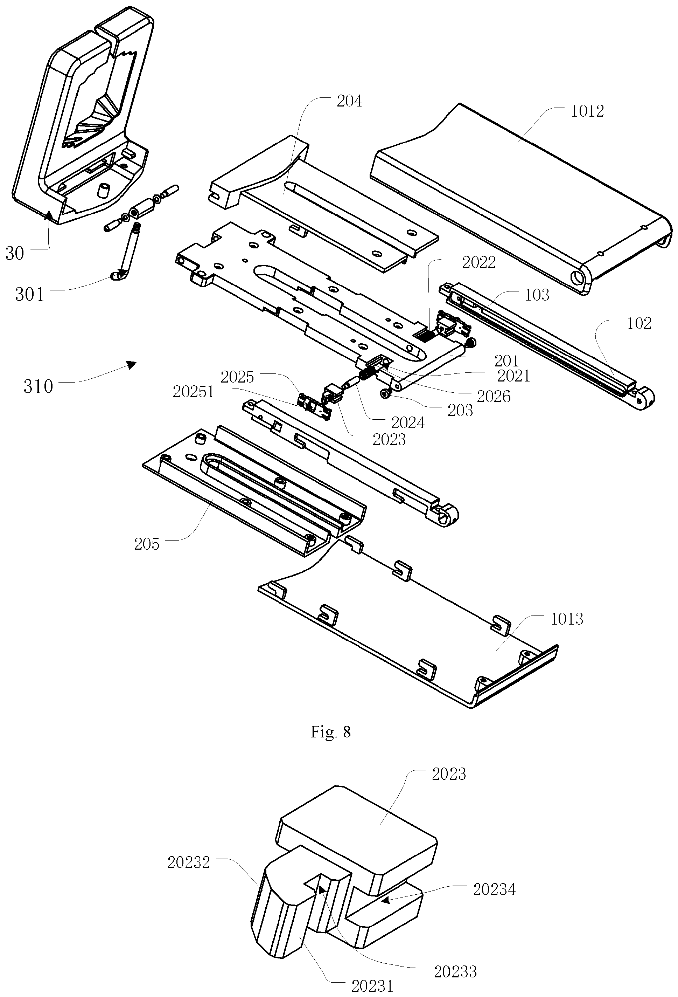

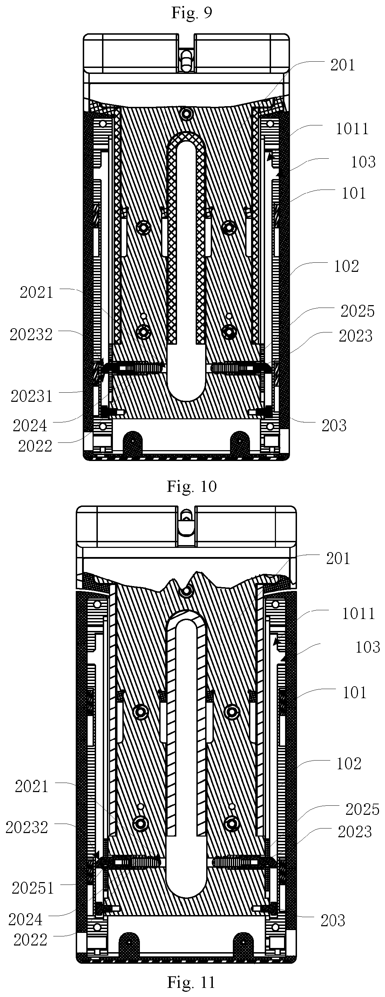

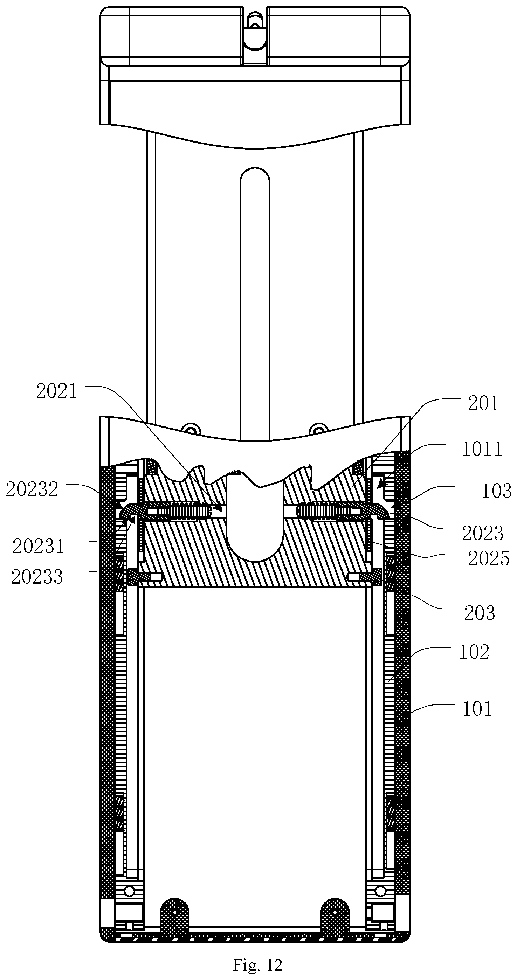

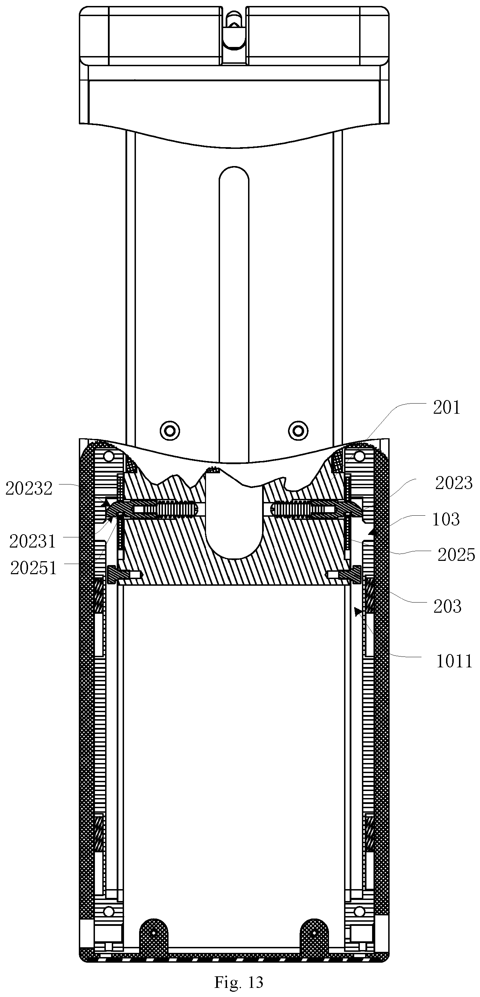

In an embodiment, the positioning assembly 202 includes a resisting buckle 2025 arranged on the sliding plate 201, a mounting hole 2021 arranged on the sliding plate 201 and communicating with the sliding space 1011, and a first spring 2022 and sliding block 2023 both installed in the mounting hole 2021. One end of the sliding block 2023 abuts against the first spring 2022, and the other end of the sliding block 2023 extends into the sliding space 1011 from the mounting hole 2021. One side of the sliding block 2023 near the first end of the slide rail 102 is provided with a resisting surface 20231; one side of the sliding block 2023 near the second end of the slide rail 102 is provided with a guide surface 20232. The first spring 2022 is in a compressed state. In this way, as shown in Fig. 12, when the sliding block 2023 slides to be opposite to the positioning hole 103, under the elastic force of the first spring 2022, the sliding block 2023 will extend into the positioning hole 103 to catch the sliding plate 201 and lock it. In this state, the sliding block 2023 is provided with a resisting surface 20231 on the side near the first end of the slide rail 102 (i.e., the lower end of the slide rail 102 shown in Fig. 10). In this way, as shown in Fig. 12, when the sliding plate 201 is pushed toward the first end of the slide rail 102, because the resisting surface 20231 of the sliding block 2023 abuts against the inner wall of the positioning hole 103, the sliding plate 201 cannot move. Since the side of the sliding block 2023 near the second end of the slide rail 102 (i.e., the upper end of the slide rail 102 shown in Fig. 10) is provided with a guide surface 20232 (alternatively, the guide surface 20232 is an inclined surface or an arc surface, as long as the sliding block 2023 can slide out along the guide surface 20232 when the sliding plate 201 is pushed toward the second end of the slide rail 102). In this state, if the sliding plate 201 is pushed toward the second end of the slide rail 102, under the guidance of the guide surface 20232, the sliding block 2023 can gradually slide out of the positioning hole 103 along the guide surface 20232 as shown in Fig. 13, thus unlocking the sliding plate 201. Furthermore, a guide rail 2026 perpendicular to the slide rail 102 is arranged on the inner side wall of the mounting hole 2021, and a guide groove 20234 slidably connected with the guide rail 2026 is arranged on the sliding block 2023. That is, the sliding block 2023 moves along the guide rail 2026 via the guide groove 20234, which can ensure the accuracy of the moving direction. In an embodiment, as shown in Figs. 8 and 10, the positioning assembly 202 further includes a positioning pin 2024 connected to the sliding block 2023, and the first spring 2022 is sleeved at one end of the positioning pin 2024 away from the sliding block 2023. That is, the positioning pin 2024 is fixed on the sliding block 2023 via threaded connection, and the positioning pin 2024 passes through the first spring 2022 and extends into a pin hole (not shown) provided on the sliding plate 201. The arrangement of the positioning pin 2024 can ensure that the first spring 2022 does not deform during the extension and retraction, thus ensuring the stability of its elasticity.

The resisting buckle 2025 is provided with a through hole 2025, and the sliding block 2023 extends into the sliding space 1011 from the mounting hole 2021 through the through hole 2025. A clamping groove 20233 adapted to the resisting buckle 2025 is formed on the resisting surface 20231 of the sliding block 2023. As shown in Figs. 8 and 10, a resisting buckle 2025 arranged on the sliding plate 201 is slidably connected with the slide rail 102. After the sliding block 2023 extends into the sliding space 1011 from the mounting hole 2021 through the through hole 2025, the clamping groove 20233 can be clamped with the resisting buckle 2025, but they may not be clamped. As shown in Fig. 10, the first spring 2022 is in a compressed state when the clamping groove 20233 is clamped with the resisting buckle 2025. Understandably, when the clamping groove 20233 is clamped with the resisting buckle 2025, the sliding block 2023 is in contact with the inner side wall of the slide rail 102. When the sliding plate 201 is completely retracted in the sliding space 1011, the clamping groove 20233 and the resisting buckle 2025 are in a clamping state.

Furthermore, in the direction parallel to the slide rail 102, the width of the through hole 2025 is greater than the maximum width of the relative moving segment of the sliding block 2023. The relative moving segment includes a first length segment opposite to the clamping groove 20233, and a second length segment connected with the first length segment and wider than the first length segment. That is, the relative moving segment refers to the length segment that the sliding block 2023 needs to pass through the through hole 2025 when it moves in the mounting hole 2021 (including the length that the sliding block 2023 needs to move in the through hole 2025 when it extends into or slides out of the positioning hole 103). The width of the through hole 2025 needs to allow the sliding block 2023 to move in the through hole 2025, so that it can switch between the two states of clamping and not clamping with the resisting buckle 2025. Specifically, as shown in fig. 11, when the sliding plate 201 needs to be stretched, the sliding plate 201 is pulled upward. In this state, the sliding block 2023 moves upward in the through hole 2025, and the clamping groove 20233 and the resisting buckle 2025 switch from the clamping state to the non-clamping state. In this state, if the sliding plate 201 is continuously pulled upward, then when the sliding plate 201 slides along the slide rail 102 to a position where the positioning assembly 202 is opposite to the positioning hole 103, the first spring 2022, which is still in compression, will continue to push the sliding block 2023 into the positioning hole 103 by its elastic force, and then lock the sliding plate 201 in the positioning hole 103 as shown in Fig. 12.

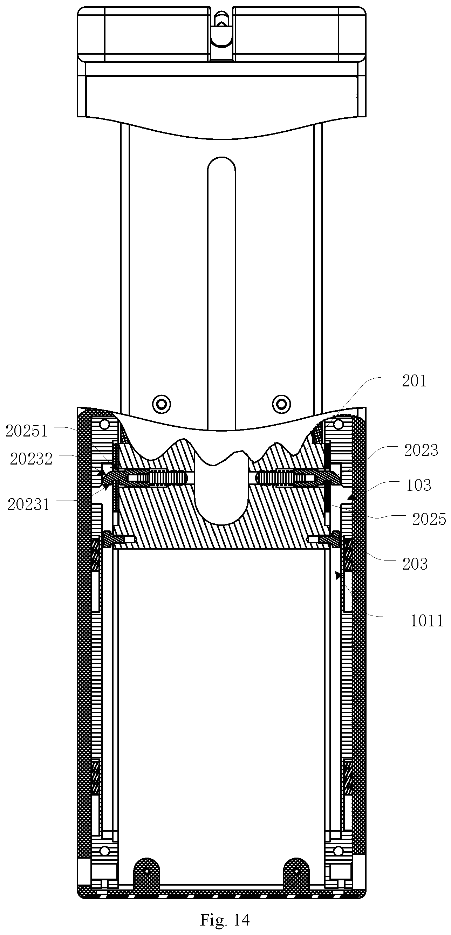

Understandably, as shown in Fig. 12, the sliding plate 201 is locked. And as shown in Fig. 13, after the sliding plate 201 is continuously pulled upward to lead the sliding plate 201 out of the positioning hole 103 via the guide surface 20232, so as to unlock the sliding plate 201, in this state, since the sliding block 2023 is in contact with the inner wall of the slide rail 102, and the clamping groove 20233 is opposite to the resisting buckle 2025, if the sliding plate 201 is pushed down. Then as shown in Fig. 14, the clamping groove 20233 and the resisting buckle 2025 switch from the non-clamping state to the clamping state, so when the sliding plate 201 is pushed downwards, even if it moves to the position where the sliding block 2023 is opposite to the positioning hole 103, because the clamping groove 20233 is locked on the resisting buckle 2025 and cannot move towards the positioning hole 103, the sliding plate 201 can continue to move downwards without being locked by the positioning hole 103, and finally the sliding plate 201 can be completely retracted in the sliding space 1011 (as shown in Fig. 10).

In a specific embodiment, the locking process of the telescopic structure 310 is as follows.

When the sliding plate 201 of the telescopic structure 310 is completely retracted in the sliding space 1011, that is, the telescopic structure 310 is in an unextended state, as shown in Fig. 10. In this state, the clamping groove 20233 is clamped with the resisting buckle 2025. When the telescopic structure 310 needs to be extended, the sliding plate 201 is pulled upward. In this state, the sliding plate 201 moves upward (the resisting bucket 2025 does not move in this state). When the sliding plate 201 moves upward by a certain distance (for example, about 1.5 mm), the sliding block 2023 will touch the resisting bucket 2025. In this state, the clamping groove 20233 and resisting buckle 2025 are no longer clamped, and then the sliding block 2023 drives the resisting buckle 2025 to move upward together. In this state, the sliding block 2023 tends to move outward under the elastic force of first spring 2022 in the compressed state (as shown in Fig. 11). When the sliding plate 201 continues to be stretched upward to a preset length (for example, 100 mm), the sliding block 2023 is opposite to the positioning hole 103. In this state, the sliding block 2023 will quickly enter the positioning hole 103 under the elastic force of the first spring 2022, with a sound of "click". This indicates that the sliding plate 201 has been locked by the positioning hole 103. In this state, the telescopic structure 310 is in the extended and self-locking state, and the downward sliding will be hindered (as shown in Figure 12).

The unlocking process of the telescopic structure 310 is as follows.

When the telescopic structure 310 is in the extended state (as shown in Fig. 12) and in the self-locking state, it cannot move downward. In this state, when the sliding plate 201 is pulled upward, the guide surface 20232 of the sliding block 2023 will convert the pulling force into a thrust force that pushes the sliding block 2023 to further compress the first spring 2022 (the thrust force direction is opposite to the elastic force direction of the first spring 2022). In this way, under the guidance of the guide surface 20232 which generates thrust, the sliding block 2023 can gradually slide out of the positioning hole 103 along the guide surface 20232 as shown in Fig. 13, thus unlocking the sliding plate 201. In this state, when the sliding plate 201 is pushed back for a short distance, the resisting buckle 2025 will be clamped into the clamping groove 20233 under the sliding block 2023 (as shown in Figure 14), and the sliding block 2023 will not slide outward or inward due to the blocking of the resisting buckle 2025. In this state, the sliding plate 201 can be pushed back to the position shown in Fig. 10 smoothly.

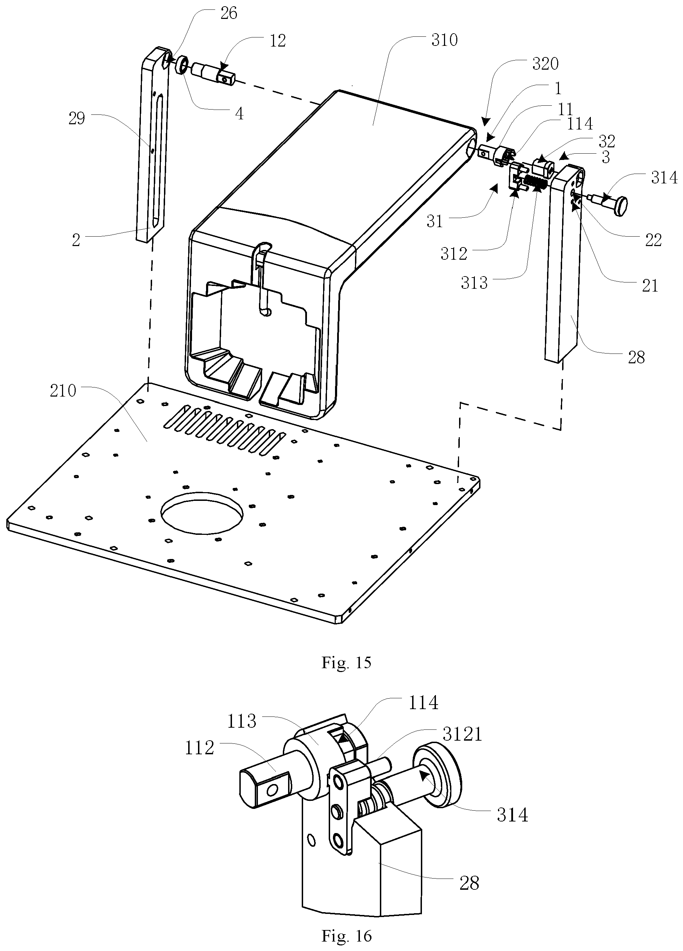

In an embodiment, as shown in Figs. 15 to 20, the rotating self-locking structure 320 includes the following components.

A rotating assembly 1, which includes a first rotating shaft 11 and a second rotating shaft 12. The first rotating shaft 11 and second rotating shaft 12 are fixedly installed on opposite sides of the support plate 101 and coaxially arranged. Specifically, both the first rotating shaft 11 and second rotating shaft 12 are fixedly connected with the support plate 101 of the telescopic structure, for example, by means of screw connection.

A bracket 2; the second rotating shaft 12 is rotatably connected to the bracket 2. In an embodiment, the bracket 2 is provided with a bearing hole 26, and the rotating assembly 1 further comprises a bearing 4, and the second rotating shaft 12 is rotatably installed in the bearing hole 26 via the bearing 4. That is, the bearing 4 is tightly fixed in the bearing hole 26, and the second rotating shaft 12 is tightly fixed in the inner ring of the bearing 4 and can rotate freely. Furthermore, as shown in Fig. 1, the bracket 2 includes a first support plate 28 and a second support plate 29, both installed on the bottom plate 210. The second rotating shaft 12 is rotatably mounted on the second support plate 29, and the first rotating shaft 11 is rotatably mounted on the first support plate 28 via the torque hinge 32. That is, one side of the support plate 101 is rotatably connected to the second support plate 29 via the second rotating shaft 12, the other side of the support plate 101 is rotatably connected to the first support plate 28 via the first rotating shaft 11 and torque hinge 32, thus the support plate 101 can rotate relative to the bracket 2 together with the first rotating shaft 11 and second rotating shaft 12.

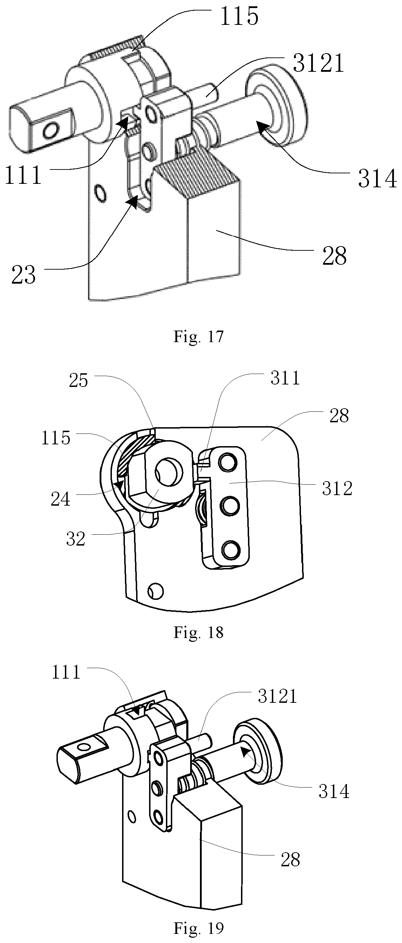

A lock assembly 3, which includes an elastic self-locking member 31 slidably connected with the bracket 2 and a torque hinge 32 installed on the bracket 2. The first rotating shaft 11 is rotatably connected with the bracket 2 via the torque hinge 32. The first rotating shaft 11 is provided with a positioning groove 111, and the elastic self-locking member 31 is provided with a locking column 311 adapted to the positioning groove 111. The support plate 101 rotates around the first rotating shaft 11 relative to the bottom plate 210 within a preset angle range. The locking column 311 is used to slide into the positioning groove 111 when the support plate 101 rotates around the first rotating shaft 11 to a preset position, thereby locking the support plate 101 to limit the rotation of the support plate 101 around the first rotating shaft 11, and the locking column 311 is also used to unlock the support plate 101 when sliding out of the positioning groove 111. As shown in Fig. 16, the locking column 311 is positioned in the positioning groove 111. In this state, the first rotating shaft 11 is blocked by the locking column 311 and cannot rotate. Therefore, the support plate 101 does not need to rotate with the first rotating shaft 11, so the support plate 101 is locked. As shown in Fig. 17, the locking column 311 slides out along the positioning groove 111. When the locking column 311 completely slides out of the positioning groove 111, the first rotating shaft 11 is no longer clamped by the locking column 311. In this state, the first rotating shaft 11 can drive the support plate 101 to rotate relative to the bracket 2.

In this embodiment, the rotating self-locking structure 320 can realize the self-locking and folding of the support plate 101 (I.e., the telescopic structure). That is, the support plate 101 can rotate and self-lock between the working position and non-working position via the above-mentioned rotating self-locking structure 320, so that the telescopic structure can be locked at the working position, and then the normal operations such as liquid transfer can be performed, thus ensuring the structural stability of the liquid transfer device during the operations such as liquid transfer. When there is no need for operations such as liquid transfer, the telescopic structure is locked and folded in the non-working position by rotating, thus reducing the volume of the whole liquid transfer device. The rotating self-locking structure 320 of the present application can realize the folding and self-locking of the telescopic structure within a preset angle range (such as an angle range of 0°-90°). The rotating self-locking structure 320 of the present application improves the portability and structural stability of the liquid transfer device, improves its moving convenience while ensuring the normal liquid transfer operation, and is also convenient for users to use it for liquid transfer operation in a small space.

In an embodiment, as shown in Figs. 15 to 20, the elastic self-locking member 31 includes a lock buckle 312, a second spring 313 and an unlocking pin 314. The locking column 311 is arranged on the lock buckle 312; the bracket 2 is provided with a first sliding hole 21 and a second sliding hole 22 which are parallel to each other. The lock bucket 312 is provided with a sliding column 3121 that passes through the first sliding hole 21 from the first side of the bracket 2 and slides along it, and the unlocking pin 314 passes through the second sliding hole 22 from the second side of the bracket 2 away from the first side and is connected with the lock bucket 312. The second spring 313 is sleeved on the unlocking pin 314 and connected between the bracket 2 and the lock buckle 312 via compression. Specifically, the number of the first sliding holes 21 can be determined as required. For example, the number of the sliding columns 3121 on the lock buckle 312 shown in Fig. 15 is two. In this state, the number of the first sliding holes 21 needs to be set to two accordingly, and all the first sliding holes 21 are parallel to each other. That is, the sliding column 3121 of the lock buckle 312 passes through the first sliding hole 21 from the direct first side, and the unlocking pin 314 passes through the second sliding hole 22 on the bracket 2 from the second side. And the end of the unlocking pin 314 passing through the second sliding hole 22 is fixed on the lock buckle 312 after the second spring 313 is sleeved, so that the lock buckle 312 and the unlocking pin 314 are fixed into a whole. Since the first sliding hole 21 and second sliding hole 22 are parallel, when the unlocking pin 314 slides along the second sliding hole 22, the sliding column 3121 will also drive the lock buckle 312 to slide along the first sliding hole 21. Furthermore, the locking column 311 arranged on the lock buckle 312 will also move in the direction parallel to the first sliding column 3121. Therefore, in this application, the positioning groove 111 (the locking column 311 slides along the positioning groove 111) and the first sliding hole 21 also need to be arranged in parallel. As shown in Fig. 16, the locking column 311 is positioned in the positioning groove 111. In this state, the first rotating shaft 11 is clamped by the locking column 311 and cannot rotate. Therefore, the support plate 101 does not need to rotate with the first rotating shaft 11, so the support plate 101 is locked. When unlocking the support plate 101, as shown in Fig. 17, the unlocking pin 314 is pulled away from the bracket 2. In this state, the unlocking pin 314 slides along the second sliding hole 22, thereby driving the sliding column 3121 and the lock buckle 312 to slide along the first sliding hole 21, which further drives the locking column 311 on the lock buckle 312 to slide out along the positioning groove 111. When the locking column 311 completely slides out of the positioning groove 111, the first rotating shaft 11 is no longer clamped by the locking column 311. In this state, the first rotating shaft 11 can drive the support plate 101 to rotate relative to the bracket 2.

In an embodiment, as shown in Fig. 17, the bracket 2 is provided with a sliding groove 23 adapted to the lock buckle 312. The lock buckle 312 is slidably installed in the sliding groove 23. That is, the shape of the sliding groove 23 is adapted to the outer contour of the lock bucket 312, so that the lock bucket 312 can maintain its stability during sliding. The lock buckle 312 in Fig. 17 is similar to a square (there is a protruding locking column 311 on the square), so the sliding groove 23 is also similar to a square. During the sliding of the sliding column 3121 along the first sliding hole 21, the lock buckle 312 itself will also slide along the sliding groove 23, thus the sliding direction of the lock buckle 312 along the sliding groove 23 is parallel to the first sliding hole 21.

In an embodiment, the lock buckle 312 is provided with a groove (not shown) adapted to the second spring 313, and the bottom of the groove is provided with a connecting hole (not shown). The second spring 313 is placed in the groove, and the unlocking pin 314 passes through the second spring 313 and is connected with the connecting hole. That is, in this embodiment, the second spring 313 is placed in a groove on the lock buckle 312, and the groove is preferably a circular groove adapted to the second spring 313. The unlocking pin 314 passes through the second sliding hole 22 (clearance fit with the second sliding hole 22) and the second spring 313, and is fixed in the connecting hole via threaded connection.

In an embodiment, as shown in Figs. 16 to 20, the first rotating shaft 11 includes a connecting segment 112 connected to the support plate 101 and a cooperating segment 113 connected to the connecting segment 112. The cooperating segment 113 is provided with an accommodating groove 114, the bracket 2 is provided with an accommodating hole 24, one end of the torque hinge 32 is interference fitted in the accommodating hole 24, and the other end of the torque hinge 32 is interference fitted in the accommodating groove 114. Specifically, one end of the torque hinge 32 is fixed in the accommodating groove 114 of the first rotating shaft 11 by interference fit, and the other end of the torque hinge 32 is fixed in the accommodating hole 24 of the bracket 2 by interference fit. Moreover, the cooperating segment 113 of the first rotating shaft 11 is inserted into the accommodating hole 24 of the bracket 2(first support plate 28) and fits with the accommodating hole 24 in clearance, so that the first rotating shaft 11 can rotate in the accommodating hole 24.

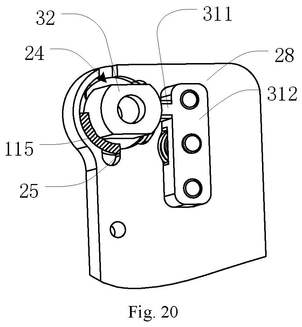

In an embodiment, as shown in Figs. 18 and 20, the cooperating segment 113 is provided with an arc baffle 115, which is inserted into the accommodating hole 24 and slidably connected between the torque hinge 32 and the inner side wall of the accommodating hole 24. A stop block 25 is arranged on the inner side wall of the accommodating hole 24. The stop block 25 is used to restrict the first rotating shaft 11 from driving the support plate 101 to rotate when it abuts against the arc baffle 115. That is, the arc buffer 115 is inserted into the accommodating hole 24; and both the arc baffle 115 and torque hinge 32 can rotate in the accommodating hole 24, at least two stop blocks 25 can be arranged on the inner side wall of the accommodating hole 24. As shown in Fig. 18, the first rotating shaft 11 rotates clockwise until the arc baffle 115 abuts against the stop block 25 above. In this state, the first rotating shaft 11 cannot continue to rotate clockwise due to the limitation of the stop block 25. Similarly, as shown in Fig. 20, the first rotating shaft 11 rotates counterclockwise until the arc baffle 115 abuts against the stop block 25 below. In this state, the first rotating shaft 11 cannot continue to rotate counterclockwise due to the limitation of the stop block 25. The upper and lower stop block 25 in Figs. 18 and 20 are designed to be 180 degrees apart in the circumferential direction of the accommodating hole 24, and the length of the arc baffle 115 is designed to be equal to a quarter of the circumference of the accommodating hole 24. Therefore, the actual rotation angle range of the arc baffle 115 in the accommodating hole 24 is only 0 to 90 degrees. Therefore, in the embodiments shown in Figs. 18 and 20, the first rotating shaft 11 can only drive the support plate 101 to rotate 90 degrees. Understandably, in this application, if it is required to modify the rotation angle range of the support plate 101 in order to change the rotatable angle of the support plate 101, the length of the arc baffle 115 and the position arrangement of the stop block 25 can be adjusted, which is not repeated here.

In an embodiment, as shown in Figs. 16 to 20, at least two positioning grooves 111 are arranged on the cooperating segment 113. At least one of the positioning groove 111 is aligned with the locking column 311 when the stop block 25 abuts against the arc baffle 115. Specifically, the cooperating segment 113 is provided with two or more positioning grooves 111 to realize multi-level positioning of the support plate 101. In the embodiments shown in Figs. 16 to 20, only two positioning grooves 111 are provided, and as shown in Fig. 18, when the first rotating shaft 11 rotates clockwise until the arc baffle 115 abuts against the stop block 25 above, the first rotating shaft 11 cannot continue to rotate clockwise due to the limitation of the stop block 25. In this state, the locking column 311 on the lock buckle 312 will be opposite to one of the positioning groove 111. If the unlocking pin 314 is not controlled, as shown in Fig. 16, it will slide into the positioning groove 111 by itself under the pulling force of the second spring 313, and fix the support plate 101 at the rotation angle. Similarly, as shown in Fig. 20, when the first rotating shaft 11 rotates counterclockwise until the arc baffle 115 abuts against the stop block 25 below, the first rotating shaft 11 cannot continue to rotate counterclockwise due to the limitation of the stop block 25. In this state, the locking column 311 on the lock buckle 312 will be opposite to another positioning groove 111. If the unlocking pin 314 is not controlled, as shown in Fig. 19, will slide into another positioning groove 111 by itself under the pulling force of the second spring 313, so as to position the support plate 101 at another angle.

In an embodiment, the self-locking and unlocking process of the rotating self-locking structure 320 are as follows.

As shown in Fig. 16, the support plate 101 of the rotating self-locking structure 320 is in a 0° self-locking state, and the lock buckle 312 is clamped into the first positioning groove 111 of the first rotating shaft 11. In this state, the first rotating shaft 11 cannot rotate, and when the unlocking pin 314 fixed with the lock buckle 312 is pulled out, the locking column 311 will slide out along the positioning groove 111 until the positioning groove 111 slides by a preset distance (for example, 3.25 mm), the locking column 311 of the lock buckle 312 is separated from the positioning groove 111 of the first rotating shaft 11 (as shown in Figure 17). In this state, the first rotating shaft 11 can be rotated counterclockwise (clockwise, the door baffle has been blocked by the stop block 25 above and cannot be rotated, as shown in Fig. 18), until it is rotated from 0° to 90°, since the arc baffle 115 cannot be rotated counterclockwise because it is blocked by the stop block 25 below (as shown in Figure 20), the unlocking pin 314 is released in this state, and under the force of the second spring 313, the locking column 311 of the lock buckle 312 will be clamped into another positioning groove 111 of the first rotating shaft 11 (as shown in Fig. 19). In this state, the second rotating shaft 12 will be locked in this position again. The process of unlocking, rotating and re-locking in the position shown in Fig. 19 is the same, and will not be repeated here.

## Claims

1. A liquid transfer device, comprising:
a liquid path mechanism (100), comprising a conduit assembly (110) and a driving member (120) for driving liquid to flow in the conduit assembly (110);
a housing assembly (200), comprising a bottom plate (210) and a housing (220) with an accommodating space (221), wherein both the conduit assembly (110) and the driving member (120) are installed on the housing (220);
a support frame (300), comprising a rotating self-locking structure (320) and a telescopic structure (310); the telescopic structure (310) is installed on the bottom plate (210) through the rotating self-locking structure (320); the rotating self-locking structure (320) is used for driving the telescopic structure (310) to rotate relative to the bottom plate (210) within a preset angle range, and locking the telescopic structure (310) to stop rotating once it reaches a preset position; and
an electrical assembly (400), comprising a control assembly (410) installed in the accommodating space (221), and the control assembly (410) is connected with the driving member (120);
**characterized in that** the telescopic structure further comprises:
a support assembly (10), comprising a support plate (101) with a sliding space (1011), a slide rail (102) arranged on an inner side wall of the sliding space (1011), and a positioning hole (103) arranged on the slide rail (102) and communicated with the sliding space (1011);
a sliding assembly (20), comprising a sliding plate (201) arranged in the sliding space (1011) and a positioning assembly (202) arranged on the sliding plate (201), the sliding plate (201) telescopically slides along the slide rail (102); and
the positioning assembly (202) is used for locking with the positioning hole (103) when the sliding plate (201) slides towards a first end of the slide rail (102), so that the sliding plate (201) stops sliding, and the positioning assembly (202) is also used for unlocking from the positioning hole (103) when the sliding plate (201) slides towards a second end of the slide rail (102).

2. The liquid transfer device of claim 1, wherein the liquid path mechanism (100) is further installed at a bubble sensor (130) on the housing (220), and the bubble sensor (130) is used for detecting a liquid flow state in the conduit assembly (110).

3. The liquid transfer device of claim 1, wherein the electrical assembly (400) further comprises a touch screen (450) installed on the housing (220) and connected with the control assembly (410); and/or
the electrical assembly (400) further comprises a switch button (420) installed on the housing (220) and connected with the control assembly (410).

4. The liquid transfer device of claim 1, wherein the electrical assembly (400) further comprises a battery (430) connected with the control assembly (410) and a power supply interface (440) connected with the battery (430) and the control assembly (410).

5. The liquid transfer device of claim 1, wherein the housing (220) comprises a main device housing (222) installed on the bottom plate (210) and having an opening, and a back plate (223) installed at the opening position of the main device housing (222) and connected with the bottom plate (210); the main device housing (222), the back plate (223) and the bottom plate (210) enclose to form the accommodation space, and the control assembly (410) is installed on the bottom plate (210) and positioned in the accommodation space.

6. The liquid transfer device of claim 1, wherein the positioning assembly (202) comprises a resisting buckle (2025) arranged on the sliding plate (201), a mounting hole (2021) arranged on the sliding plate (201) and communicated with the sliding space (1011), and a first spring (2022) and a sliding block (2023) both installed in the mounting hole (2021); an end of the sliding block (2023) abuts against the first spring (2022), and another end of the sliding block (2023) extends into the sliding space (1011) from the mounting hole (2021); a side of the sliding block (2023) near the first end of the slide rail (102) is provided with a resisting surface (20231); a side of the sliding block (2023) near the second end of the slide rail (102) is provided with a guide surface (20232); and
the resisting buckle (2025) is provided with a through hole, and the sliding block (2023) passes through the through hole and extends into the sliding space (1011) from the mounting hole (2021); the resisting surface (20231); of the sliding block (2023) is recessed, forming a clamping groove (20233) adapted to the resisting buckle (2025).

7. The liquid transfer device of claim 1, wherein the rotating self-locking structure (320) comprises:
a rotating assembly, comprising a first rotating shaft (11) and a second rotating shaft (12), the first rotating shaft (11) and the second rotating shaft (12) are respectively fixedly arranged on the opposite sides of the support plate (101) and coaxially arranged;
a bracket (2), the second rotating shaft (12) is rotatably connected to the bracket (2);
a lock assembly (3), comprising an elastic self-locking member (31) slidably connected with the bracket (2) and a torque hinge (32) installed on the bracket (2), and the first rotating shaft (11) is rotationally connected with the bracket (2) via the torque hinge (32); the first rotating shaft (11) is provided with a positioning groove (111), and the elastic self-locking member is provided with a locking column (331) adapted to the positioning groove (111); the support plate (101) rotates relative to the bottom plate (210) around the first rotating shaft (11) within a preset angle range; and
the locking column (311) is used for sliding into the positioning groove (111) when the support plate (101) rotates around the first rotating shaft (11) to a preset position, so as to lock the support plate (101) to limit the rotation of the support plate (101) around the first rotating shaft (11), and the locking column (311) is also used for unlocking the support plate (101) when sliding out of the positioning groove (111).

8. The liquid transfer device of claim 7, wherein the elastic self-locking member (31) comprises a lock buckle (312), a second spring (313) and an unlocking pin (314); the locking column (311) is arranged on the lock buckle (312); the bracket (2) is provided with a first sliding hole (21) and a second sliding hole (22) parallel to each other, and the lock buckle (312) is provided with a sliding column (3121), the sliding column (3121) passes through and slides along the first sliding hole (21) from a first side of the bracket (2); the unlocking pin (314) passes through the second sliding hole (22) from a second side of the bracket (2) away from the first side and is connected with the lock buckle (312); and the second spring (313) is sleeved on the unlocking pin (314) and pressed between the bracket (2) and the lock buckle (312).

9. The liquid transfer device of claim 7, wherein the first rotating shaft comprises a connecting segment (112) connected with the support plate (101) and a cooperating segment (113) connected with the connecting segment (112); the cooperating segment is provided with an accommodating groove (114), the bracket (2) is provided with an accommodating hole (24), an end of the torque hinge (32) is interference-fitted in the accommodating hole (24), and another end of the torque hinge (32) is interference-fitted in the accommodating groove (114);
the cooperating segment (113) is provided with an arc baffle (115), the arc baffle (115) is inserted into the accommodating hole (24) and slidably connected between the torque hinge (32) and an inner side wall of the accommodating hole (24); a stop block (25) is arranged on the inner side wall of the accommodating hole (24); the stop block (25) is used for limiting the first rotating shaft (11) to drive the support plate to rotate when abutting against the arc baffle (115); and
at least two positioning grooves (111) are arranged on the cooperating segment (113); at least one positioning groove (111) is aligned with the locking column (311) when the stop block (25) abuts against the arc baffle (115).

## Patentansprüche

1. Eine Flüssigkeitsübertragungsvorrichtung, umfassend:
eine Flüssigkeitswegmechanik (100), umfassend eine Leitungsanordnung (110) und ein Antriebselement (120) zum Antreiben eines Flüssigkeitsstroms in der Leitungsanordnung (110);
eine Gehäuseanordnung (200), umfassend eine Bodenplatte (210) und ein Gehäuse (220) mit einem Aufnahmeraum (221), wobei sowohl die Leitungsanordnung (110) als auch das Antriebselement (120) an dem Gehäuse (220) angebracht sind;
einen Tragrahmen (300), umfassend eine drehbare Selbstsicherungsstruktur (320) und eine Teleskopstruktur (310); wobei die Teleskopstruktur (310) über die drehbare Selbstsicherungsstruktur (320) an der Bodenplatte (210) angebracht ist; wobei die drehbare Selbstsicherungsstruktur (320) dazu dient, die Teleskopstruktur (310) relativ zu der Bodenplatte (210) innerhalb eines vorgegebenen Winkelbereichs zu drehen und die Teleskopstruktur (310) zu verriegeln, sodass diese aufhört, sich zu drehen, sobald sie eine vorgegebene Position erreicht; und
eine elektrische Anordnung (400), umfassend eine in dem Aufnahmeraum (221) angebrachte Steueranordnung (410), wobei die Steueranordnung (410) mit dem Antriebselement (120) verbunden ist,
**dadurch gekennzeichnet, dass** die Teleskopstruktur ferner Folgendes umfasst:
eine Traganordnung (10), umfassend eine Trägerplatte (101) mit einem Gleitraum (1011), eine an einer Innenwand des Gleitraums (1011) angeordnete Gleitschiene (102) und ein an der Gleitschiene (102) angeordnetes und mit dem Gleitraum (1011) in Verbindung stehendes Positionierungsloch (103);
eine Schiebeanordnung (20), umfassend eine in dem Gleitraum (1011) angeordnete Schiebeplatte (201) und eine an der Schiebeplatte (201) angeordnete Positionierungsanordnung (202), wobei die Schiebeplatte (201) teleskopisch entlang der Gleitschiene (102) verschiebbar ist; und
wobei die Positionierungsanordnung (202) dazu dient, mit dem Positionierungsloch (103) zu verriegeln, wenn die Schiebeplatte (201) zu einem ersten Ende der Gleitschiene (102) hin verschoben wird, sodass die Schiebeplatte (201) aufhört, sich zu verschieben, und die Positionierungsanordnung (202) ferner dazu dient, sich von dem Positionierungsloch (103) zu lösen, wenn die Schiebeplatte (201) zu einem zweiten Ende der Gleitschiene (102) hin verschoben wird.

2. Die Flüssigkeitsübertragungsvorrichtung nach Anspruch 1, wobei die Flüssigkeitswegmechanik (100) ferner mit einem an dem Gehäuse (220) angebrachten Blasensensor (130) versehen ist, wobei der Blasensensor (130) dazu dient, einen Flüssigkeitsströmungszustand in der Leitungsanordnung (110) zu erfassen.

3. Die Flüssigkeitsübertragungsvorrichtung nach Anspruch 1, wobei die elektrische Anordnung (400) ferner einen an dem Gehäuse (220) angebrachten und mit der Steueranordnung (410) verbundenen Touchscreen (450) umfasst; und/oder
die elektrische Anordnung (400) ferner eine an dem Gehäuse (220) angebrachte und mit der Steueranordnung (410) verbundene Schalttaste (420) umfasst.

4. Die Flüssigkeitsübertragungsvorrichtung nach Anspruch 1, wobei die elektrische Anordnung (400) ferner eine mit der Steueranordnung (410) verbundene Batterie (430) und eine mit der Batterie (430) und der Steueranordnung (410) verbundene Stromversorgungsschnittstelle (440) umfasst.

5. Die Flüssigkeitsübertragungsvorrichtung nach Anspruch 1, wobei das Gehäuse (220) ein auf der Bodenplatte (210) angebrachtes Hauptvorrichtungsgehäuse (222) mit einer Öffnung und eine an der Öffnung des Hauptvorrichtungsgehäuses (222) angebrachte und mit der Bodenplatte (210) verbundene Rückplatte (223) umfasst; wobei das Hauptvorrichtungsgehäuse (222), die Rückplatte (223) und die Bodenplatte (210) den Aufnahmeraum einschließen und die Steueranordnung (410) auf der Bodenplatte (210) angebracht und in dem Aufnahmeraum angeordnet ist.

6. Die Flüssigkeitsübertragungsvorrichtung nach Anspruch 1, wobei die Positionierungsanordnung (202) einen an der Schiebeplatte (201) angeordneten Anschlagbügel (2025), eine an der Schiebeplatte (201) angeordnete Montageöffnung (2021), die mit dem Gleitraum (1011) in Verbindung steht, sowie eine erste Feder (2022) und einen Gleitblock (2023) umfasst, die beide in der Montageöffnung (2021) angeordnet sind; wobei ein Ende des Gleitblocks (2023) an der ersten Feder (2022) anliegt und sich das andere Ende des Gleitblocks (2023) von der Montageöffnung (2021) aus in den Gleitraum (1011) erstreckt; wobei eine dem ersten Ende der Gleitschiene (102) zugewandte Seite des Gleitblocks (2023) mit einer Anschlagfläche (20231) versehen ist; wobei eine dem zweiten Ende der Gleitschiene (102) zugewandte Seite des Gleitblocks (2023) mit einer Führungsfläche (20232) versehen ist; und
wobei der Anschlagbügel (2025) mit einer Durchgangsöffnung versehen ist, wobei der Gleitblock (2023) durch die Durchgangsöffnung hindurchgeführt ist und sich von der Montageöffnung (2021) aus in den Gleitraum (1011) erstreckt; wobei die Anschlagfläche (20231) des Gleitblocks (2023) vertieft ausgebildet ist, wodurch eine an den Anschlagbügel (2025) angepasste Eingriffsnut (20233) gebildet wird.

7. Die Flüssigkeitsübertragungsvorrichtung nach Anspruch 1, wobei die drehbare Selbstsicherungsstruktur (320) Folgendes umfasst:
eine Drehanordnung, umfassend eine erste Drehwelle (11) und eine zweite Drehwelle (12), wobei die erste Drehwelle (11) und die zweite Drehwelle (12) jeweils fest an gegenüberliegenden Seiten der Trägerplatte (101) angeordnet und koaxial zueinander angeordnet sind;
einen Halter (2), wobei die zweite Drehwelle (12) drehbar mit dem Halter (2) verbunden ist;
eine Verriegelungsanordnung (3), umfassend ein elastisches Selbstsicherungselement (31), das verschiebbar mit dem Halter (2) verbunden ist, und ein Drehmomentscharnier (32), das an dem Halter (2) angebracht ist, wobei die erste Drehwelle (11) über das Drehmomentscharnier (32) drehbar mit dem Halter (2) verbunden ist; wobei die erste Drehwelle (11) mit einer Positionierungsnut (111) versehen ist und das elastische Selbstsicherungselement mit einer an die Positionierungsnut (111) angepassten Verriegelungssäule (311) versehen ist; wobei sich die Trägerplatte (101) relativ zu der Bodenplatte (210) um die erste Drehwelle (11) innerhalb eines vorgegebenen Winkelbereichs dreht; und
wobei die Verriegelungssäule (311) dazu dient, in die Positionierungsnut (111) zu gleiten, wenn die Trägerplatte (101) sich um die erste Drehwelle (11) in eine vorgegebene Position dreht, um dadurch die Trägerplatte (101) zu verriegeln und die Drehung der Trägerplatte (101) um die erste Drehwelle (11) zu begrenzen, und wobei die Verriegelungssäule (311) ferner dazu dient, die Trägerplatte (101) zu entriegeln, wenn sie aus der Positionierungsnut (111) herausgleitet.

8. Die Flüssigkeitsübertragungsvorrichtung nach Anspruch 7, wobei das elastische Selbstsicherungselement (31) einen Verriegelungsbügel (312), eine zweite Feder (313) und einen Entriegelungsstift (314) umfasst; wobei die Verriegelungssäule (311) an dem Verriegelungsbügel (312) angeordnet ist; wobei der Halter (2) mit einem ersten Gleitloch (21) und einem zweiten Gleitloch (22) versehen ist, die parallel zueinander verlaufen, und der Verriegelungsbügel (312) mit einer Gleitsäule (3121) versehen ist, wobei die Gleitsäule (3121) von einer ersten Seite des Halters (2) aus durch das erste Gleitloch (21) hindurchgeführt ist und entlang diesem gleitet; wobei der Entriegelungsstift (314) von einer der ersten Seite abgewandten zweiten Seite des Halters (2) aus durch das zweite Gleitloch (22) hindurchgeführt und mit dem Verriegelungsbügel (312) verbunden ist; und wobei die zweite Feder (313) auf den Entriegelungsstift (314) aufgesteckt ist und zwischen dem Halter (2) und dem Verriegelungsbügel (312) zusammengedrückt ist.

9. Die Flüssigkeitsübertragungsvorrichtung nach Anspruch 7, wobei die erste Drehwelle einen mit der Trägerplatte (101) verbundenen Verbindungsabschnitt (112) und einen mit dem Verbindungsabschnitt (112) verbundenen Zusammenwirkabschnitt (113) umfasst; wobei der Zusammenwirkabschnitt mit einer Aufnahmeausnehmung (114) versehen ist, der Halter (2) mit einer Aufnahmeöffnung (24) versehen ist, ein Ende des Drehmomentscharniers (32) mit Presssitz in die Aufnahmeöffnung (24) eingesetzt ist und das andere Ende des Drehmomentscharniers (32) mit Presssitz in die Aufnahmeausnehmung (114) eingesetzt ist;
wobei der Zusammenwirkabschnitt (113) mit einem bogenförmigen Anschlag (115) versehen ist, wobei der bogenförmige Anschlag (115) in die Aufnahmeöffnung (24) eingesetzt und zwischen dem Drehmomentscharnier (32) und einer Innenwand der Aufnahmeöffnung (24) gleitbar verbunden ist; wobei ein Anschlagblock (25) an der Innenwand der Aufnahmeöffnung (24) angeordnet ist; wobei der Anschlagblock (25) dazu dient, die erste Drehwelle (11) daran zu hindern, die Trägerplatte zum Drehen anzutreiben, wenn er an dem bogenförmigen Anschlag (115) anliegt; und
wobei mindestens zwei Positionierungsnuten (111) an dem Zusammenwirkabschnitt (113) angeordnet sind; wobei mindestens eine Positionierungsnut (111) mit der Verriegelungssäule (311) ausgerichtet ist, wenn der Anschlagblock (25) an dem bogenförmigen Anschlag (115) anliegt.

## Revendications

1. Un dispositif de transfert de liquide, comprenant :
un mécanisme de circulation de liquide (100), comprenant un ensemble de conduits (110) et un élément d'entraînement (120) destiné à entraîner un liquide à s'écouler dans l'ensemble de conduits (110) ;
un ensemble de boîtier (200), comprenant une plaque inférieure (210) et un boîtier (220) présentant un espace de logement (221), dans lequel l'ensemble de conduits (110) et l'élément d'entraînement (120) sont tous deux montés sur le boîtier (220) ;
un cadre de support (300), comprenant une structure rotative d'auto-verrouillage (320) et une structure télescopique (310) ; la structure télescopique (310) étant montée sur la plaque inférieure (210) par l'intermédiaire de la structure rotative d'auto-verrouillage (320) ; la structure rotative d'auto-verrouillage (320) étant destinée à entraîner la structure télescopique (310) en rotation par rapport à la plaque inférieure (210) dans une plage angulaire prédéfinie, et à verrouiller la structure télescopique (310) de manière à empêcher celle-ci de continuer à tourner une fois qu'elle a atteint une position prédéfinie ; et
un ensemble électrique (400), comprenant un ensemble de commande (410) monté dans l'espace de logement (221), l'ensemble de commande (410) étant relié à l'élément d'entraînement (120),
**caractérisé en ce que** la structure télescopique comprend en outre :
un ensemble de support (10), comprenant une plaque de support (101) présentant un espace de coulissement (1011), un rail de guidage (102) agencé sur une paroi latérale intérieure de l'espace de coulissement (1011), et un trou de positionnement (103) agencé sur le rail de guidage (102) et communiquant avec l'espace de coulissement (1011) ;
un ensemble coulissant (20), comprenant une plaque coulissante (201) agencée dans l'espace de coulissement (1011) et un ensemble de positionnement (202) agencé sur la plaque coulissante (201), la plaque coulissante (201) coulissant de manière télescopique le long du rail de guidage (102) ; et
l'ensemble de positionnement (202) étant destiné à se verrouiller dans le trou de positionnement (103) lorsque la plaque coulissante (201) coulisse vers une première extrémité du rail de guidage (102), de sorte que la plaque coulissante (201) cesse de coulisser, et l'ensemble de positionnement (202) étant également destiné à se déverrouiller du trou de positionnement (103) lorsque la plaque coulissante (201) coulisse vers une deuxième extrémité du rail de guidage (102).

2. Le dispositif de transfert de liquide selon la revendication 1, dans lequel le mécanisme de circulation de liquide (100) est en outre pourvu d'un capteur de bulles (130) monté sur le boîtier (220), le capteur de bulles (130) étant destiné à détecter un état d'écoulement du liquide dans l'ensemble de conduits (110).

3. Le dispositif de transfert de liquide selon la revendication 1, dans lequel l'ensemble électrique (400) comprend en outre un écran tactile (450) monté sur le boîtier (220) et relié à l'ensemble de commande (410) ; et/ou l'ensemble électrique (400) comprend en outre un bouton de commutation (420) monté sur le boîtier (220) et relié à l'ensemble de commande (410).

4. Le dispositif de transfert de liquide selon la revendication 1, dans lequel l'ensemble électrique (400) comprend en outre une batterie (430) reliée à l'ensemble de commande (410) et une interface d'alimentation électrique (440) reliée à la batterie (430) et à l'ensemble de commande (410).

5. Le dispositif de transfert de liquide selon la revendication 1, dans lequel le boîtier (220) comprend un boîtier principal du dispositif (222) monté sur la plaque inférieure (210) et présentant une ouverture, et une plaque arrière (223) montée au niveau de l'ouverture du boîtier principal du dispositif (222) et reliée à la plaque inférieure (210) ; le boîtier principal du dispositif (222), la plaque arrière (223) et la plaque inférieure (210) délimitant ensemble l'espace de logement, et l'ensemble de commande (410) étant monté sur la plaque inférieure (210) et positionné dans l'espace de logement.

6. Le dispositif de transfert de liquide selon la revendication 1, dans lequel l'ensemble de positionnement (202) comprend un étrier de butée (2025) agencé sur la plaque coulissante (201), un trou de montage (2021) agencé sur la plaque coulissante (201) et communiquant avec l'espace de coulissement (1011), ainsi qu'un premier ressort (2022) et un bloc coulissant (2023) tous deux montés dans le trou de montage (2021) ; une extrémité du bloc coulissant (2023) venant en butée contre le premier ressort (2022), et l'autre extrémité du bloc coulissant (2023) s'étendant dans l'espace de coulissement (1011) depuis le trou de montage (2021) ; un côté du bloc coulissant (2023) situé à proximité de la première extrémité du rail de guidage (102) étant pourvu d'une surface de butée (20231) ; un côté du bloc coulissant (2023) situé à proximité de la deuxième extrémité du rail de guidage (102) étant pourvu d'une surface de guidage (20232) ; et
l'étrier de butée (2025) étant pourvu d'un trou traversant, le bloc coulissant (2023) traversant le trou traversant et s'étendant dans l'espace de coulissement (1011) depuis le trou de montage (2021) ; la surface de butée (20231) du bloc coulissant (2023) étant formée en creux, de manière à former une rainure d'engagement (20233) adaptée à l'étrier de butée (2025).

7. Le dispositif de transfert de liquide selon la revendication 1, dans lequel la structure rotative d'auto-verrouillage (320) comprend :
un ensemble rotatif, comprenant un premier arbre rotatif (11) et un deuxième arbre rotatif (12), le premier arbre rotatif (11) et le deuxième arbre rotatif (12) étant respectivement agencés de manière fixe sur des côtés opposés de la plaque de support (101) et étant agencés coaxialement ;
un support (2), le deuxième arbre rotatif (12) étant relié de manière rotative au support (2) ;
un ensemble de verrouillage (3), comprenant un élément élastique d'auto-verrouillage (31) relié de manière coulissante au support (2) et une charnière de couple (32) montée sur le support (2), le premier arbre rotatif (11) étant relié de manière rotative au support (2) par l'intermédiaire de la charnière de couple (32) ; le premier arbre rotatif (11) étant pourvu d'une rainure de positionnement (111), et l'élément élastique d'auto-verrouillage étant pourvu d'une colonne de verrouillage (311) adaptée à la rainure de positionnement (111) ; la plaque de support (101) tournant par rapport à la plaque inférieure (210) autour du premier arbre rotatif (11) dans une plage angulaire prédéfinie ; et
la colonne de verrouillage (311) étant destinée à coulisser dans la rainure de positionnement (111) lorsque la plaque de support (101) tourne autour du premier arbre rotatif (11) jusqu'à une position prédéfinie, de manière à verrouiller la plaque de support (101) afin de limiter la rotation de la plaque de support (101) autour du premier arbre rotatif (11), et la colonne de verrouillage (311) étant également destinée à déverrouiller la plaque de support (101) lorsqu'elle coulisse hors de la rainure de positionnement (111).

8. Le dispositif de transfert de liquide selon la revendication 7, dans lequel l'élément élastique d'auto-verrouillage (31) comprend un étrier de verrouillage (312), un deuxième ressort (313) et une goupille de déverrouillage (314) ; la colonne de verrouillage (311) étant agencée sur l'étrier de verrouillage (312) ; le support (2) étant pourvu d'un premier trou de coulissement (21) et d'un deuxième trou de coulissement (22) parallèles l'un à l'autre, et l'étrier de verrouillage (312) étant pourvu d'une colonne coulissante (3121), la colonne coulissante (3121) traversant le premier trou de coulissement (21) depuis un premier côté du support (2) et coulissant le long de celui-ci ; la goupille de déverrouillage (314) traversant le deuxième trou de coulissement (22) depuis un deuxième côté du support (2) opposé au premier côté et étant reliée à l'étrier de verrouillage (312) ; et le deuxième ressort (313) étant monté autour de la goupille de déverrouillage (314) et comprimé entre le support (2) et l'étrier de verrouillage (312).

9. Le dispositif de transfert de liquide selon la revendication 7, dans lequel le premier arbre rotatif comprend un segment de liaison (112) relié à la plaque de support (101) et un segment de coopération (113) relié au segment de liaison (112) ; le segment de coopération étant pourvu d'une rainure de logement (114), le support (2) étant pourvu d'un trou de logement (24), une extrémité de la charnière de couple (32) étant montée avec un ajustement serré dans le trou de logement (24), et l'autre extrémité de la charnière de couple (32) étant montée avec un ajustement serré dans la rainure de logement (114) ;
le segment de coopération (113) étant pourvu d'un élément de butée en arc de cercle (115), l'élément de butée en arc de cercle (115) étant inséré dans le trou de logement (24) et étant relié de manière coulissante entre la charnière de couple (32) et une paroi latérale intérieure du trou de logement (24) ; un bloc de butée (25) étant agencé sur la paroi latérale intérieure du trou de logement (24)
; le bloc de butée (25) étant destiné à limiter la rotation du premier arbre rotatif (11) entraînant la plaque de support en rotation lorsqu'il vient en butée contre l'élément de butée en arc de cercle (115) ; et
au moins deux rainures de positionnement (111) étant agencées sur le segment de coopération (113) ; au moins une rainure de positionnement (111) étant alignée avec la colonne de verrouillage (311) lorsque le bloc de butée (25) vient en butée contre l'élément de butée en arc de cercle (115).
